# EUROPEAN PATENT SPECIFICATION

(11) **EP 2 445 505 B1**
(45) Date of publication and mention of the grant of the patent: **27.03.2013**
(21) Application number: 10743215.5
(22) Date of filing: 24.06.2010
(51) Int. Cl.: A61K 31/664, A61K 38/43, A61P 9/10

(54) **COMBINED USE OF CREATINE PHOSPHATE AND CREATINE PHOSPHOKINASE FOR TREATMENT OF ARTERIOSCLEROSIS**
KOMBINIERTE VERWENDUNG VON KREATINPHOSPHAT UND KREATIN-PHOSPHOKINASE ZUR BEHANDLUNG VON ARTERIOSKLEROSE
UTILISATION COMBINÉE DE CRÉATINE PHOSPHATE ET DE CRÉATINE PHOSPHOKINASE DANS LE TRAITEMENT DE L'ARTÉRIOSCLÉROSE

(30) Priority: 24.06.2009 CH 9842009
(43) Date of publication of application: 02.05.2012
(73) Proprietor: Artcell Invest Ltd., Ajeltake Island, Majura (MH)
(72) Inventor: RODIGHIERO, Giovanni, I-30033 Noale (VE) (IT)
(74) Representative: Fabiano, Piero
(86) International application number: PCT/IB2010/001516
(87) International publication number: WO 2010/150079

(56) References cited:
- US-A- 4 371 521
- KAKISHITA E ET AL: "Inhibitory effect of vitamin E (alpha -tocopherol) on spontaneous platelet aggregation in whole blood" THROMBOSIS RESEARCH, TARRYTOWN, NY, US LNKD- DOI:10.1016/0049-3848(90)90233-3, vol. 60, no. 6, 15 December 1990 (1990-12-15), pages 489-499, XP022878787 ISSN: 0049-3848 [retrieved on 1990-12-15]

## Description

### FIELD OF THE INVENTION

The present invention refers to a technique for the treatment of arteriosclerosis and particularly, to a technique for the resolution of arteriosclerotic plaque in arteries.

### STATE OF THE ART

The exact nature of atherosclerosis is still the cause of many debates and the underlying mechanism of action is not well known yet. Although it is an extremely common pathology, to date several aspects of atherosclerosis are not known in details and only hypotheses exist. However, it is established that the formation of the atheroma is a response to chronic inflammation due to the accumulation of plasma-derived lipoproteins on selected arterial sites, that is in the proximity of an arterial wall lesion, with the consequent formation of atheromatose plaques and possible circulatory complications. Macrophages, present in atherosclerotic lesions, phagocyte the plasma-derived lipoproteins and accumulate in the lesion, thus setting up a foam cell accumulation (foam macrophage cells). Additionally, many of these cells are degenerated or dead. Foam cells aggregate on the initial arterial lesion and form yellow-coloured fatty streaks. As a result of the accumulation of lipoproteins, the arterial wall lesion evolves from the initial fatty streak to a more complex and voluminous fibro-lipid or atheromatose plaque. Said plaque consists of a central atheromatose nucleus rich in lipids (atheroma) coated with a thick fibrocellular layer with precipitates of calcium. The atherosclerotic plaque impedes and sometimes arrests arterial blood flow. The plaque causes a limited hardening of the vessel wall and once formed, tends to increase within the vessel itself, progressively narrowing its calibre. Additionally, the plaque is very dangerous since, if it should break, its blood transported fragments would cause embolism, risking to occlude vessels of smaller diameter.

### DESCRIPTION OF THE INVENTION

One objective of the present invention consists in associating two active ingredients to obtain reduction of the size and thickness of an atherosclerotic plaque present in the wall of an artery of an individual.

According to an aspect the present invention provides creatine phosphate (CP) and creatine phosphokinase (CPK) for combined use in the treatment of an atherosclerotic plaque present in the wall of an artery of a patient. Further embodiments of the invention are defined in the attached dependent claims 2-10. Specifically, the association of creatine phosphate (CP) and creatine phosphokinase (CPK) for use in reduction or resolution of an arteriosclerotic plaque of an artery of a patient is envisioned.

The applicant has observed that with concomitant or sequential administration of creatine phosphate (CP) and creatine phosphokinase (CPK) a surprising efflux of lipoproteins from the arterial plaque is obtained, which determines a reduction of size and thickness of the plaque, observed already from the first administration.

In particular, combined administration of CP and CPK triggers a series of biochemical reactions determining the efflux of lipoproteins, cholesterol and triglycerides from the atherosclerotic plaque producing a substantial reduction of the size and thickness of the plaque itself or its resolution, regardless of the fact that this is at the initial or final stage of its formation.

Typically, administration of CP and CPK can occur concomitantly or sequentially so as to allow the synergism of action of the two active ingredients that shows itself with the efflux of lipoproteins, triglycerides and cholesterol from the target arteriosclerotic plaque.

An aspect of the invention concerns creatine phosphate (CP) for use in the treatment of an atherosclerotic plaque of an artery of a patient receiving concomitantly or sequentially creatine phosphokinase (CPK).

Creatine phosphate CP 2-[(amino-phosphonoimino-methyl)-methyl-amino]acetic acid is a guanidinic organic compound found in the musculature in particular the striated musculature of animals. The activity of this molecule is attributable to the presence of a phosphono-imino group, which contains a high energy bond. Typically, CP suitable in the field of the present invention, is the high energy exogenous phosphate used instead of ATP, being the latter a chemically unstable compound, quickly hydrolyzed by extracellular ATP-ases.

In addition, the regimen of the invention allows to use also high concentrations of CP without causing a direct negative inotropic effect, differently from what occurs in the presence of high concentrations of ATP. Both CP and ATP can cross the cellular membrane. The function of CP in energy stocking is fundamental being it able to form ATP through the action of CPK: ATP can be converted into cAMP through the action of the enzyme adenyl-cyclase. ATP is present everywhere in cytoplasm and nucleoplasm of cells and basically all the energy requiring physiological mechanisms of our body, obtain it from ATP, which can be used as an energetic source for almost all the cellular functions. cAMP has a multiplicity of effects within the cells activating a cascade of enzymes and triggering biochemical reactions.

Creatine phosphokinase CPK is an enzyme present in the muscular tissue as well. Specifically three forms (isoenzymes) of CPK exist, the MM form present almost exclusively in skeletal musculature, the MB form present essentially in the cardiac muscle and the BB form present essentially in brain tissue.

For example the enzyme CPK can be obtained from skeletal muscles of rabbit, or from heart of pig typically in a lyophilized form while the isoform CPK-MM is typically obtained from human skeletal musculature and provided in the form of a solution for parenteral use.

CP and CPK are two energetic molecules able to provide the necessary energy for the activation of enzymes of the atherosclerotic plaque activating the biochemical activities of the plaque itself, with immediate and beneficial lipolytic effects on triglyceride-rich lipoproteins and releasing cholesterol present in the plaque.

Uses in the therapeutic and/or prophylactic field according to the present invention are essentially based on the following biochemical reaction:

CP + ADP ↔ C + ATP CPK

The enzyme CPK catalyzes the transformation reaction of ADP (adenosin-diphosphate) into ATP (adenosin-triphosphate) with formation of creatine C. In this reaction, which at pH 7.4 typical of blood is shifted to the right, CP acting as an energy store thanks to the presence of its high energy bond, provides energy to ADP determining its conversion into ATP.

Use of the combination of exogenous CP and CPK provides the necessary energy to reactivate the enzymes of the plaque and activate the biochemical activities of the plaque itself and thus increase the levels of cAMP.

Macrophages have a multiplicity of functions and enzymes. Only recently the role of macrophages as major cells provided with secretory activity has emerged. Many of the macrophage secreted products, including enzymes, are secreted in significant amounts only after an appropriate activation. Macrophages have many enzymes involved in atherosclerosis: acyl-Coa: cholesterol acyltransferase (ACAT), neutral cholesterol esterase, oxydases, lipoprotein lipase. All these enzymes are activated by ATP. Furthermore, macrophages can secrete proteolytic enzymes, such as collagenase and elastase.

Atherosclerotic plaques contain an atheromatose soft nucleus that is unstable and is subject to break. Usually rupture occurs where the fibrous capsule is thin, at the junction between plaque and adjacent vessel wall, less unhealthy and infiltrated by foam cells. Rupture of the plaque depends on many factors. One of these factors, is proteolytic enzymes secreted by macrophages. In fact, these can quickly degrade the components of the extracellular matrix and can weaken the fibrous capsule predisposing it to rupture. The excess of cholesterol in macrophages is esterified and converted into cholesterol ester by ACAT. Cholesterol ester accumulated in the cytoplasm of macrophages is subject to be hydrolyzed by the neutral cholesterol esterase. Macrophagic foam cells contain neutral cholesterol esterase that hydrolyzes cholesterol ester with release and efflux of free cholesterol that can be removed from the cell in the presence of an acceptor in the medium. cAMP promotes the phosphorilation of cholesterol esterase by action of a cAMP dependent protein kinase: the addition of exogenous cAMP and ATP to macrophages has given a great increase (60%) to the activity of the neutral cholesterol esterase.

Concomitant or sequential administration of CP and CPK markedly increases cAMP level. LDL can be oxidized by macrophagic oxygenases. The role of oxidized LDL is controversial. Lipoprotein lipase is the most relevant enzyme in the metabolism of triglyceride-rich lipoproteins. Macrophages synthesize and secrete lipoprotein lipase in the medium and are able to act on triglyceride-rich lipoproteins through the action of lipoprotein lipase, so as to mobilize accumulated triglycerides very quickly with their consequent efflux: mobilization is hence massive and quick. This allows the macrophages to be relatively rich in cholesterol esters: lipoprotein lipase does not act, in fact, on cholesterol esters.

Using only a low-fat diet, a first quick decrease of triglycerides from the triglycerides-rich lipoproteins is observed and then a release of the content of the lipoproteins (some days) is observed, but all the lipolysis process requires more time. Triglycerides in fact are released first and subsequently the release of the lipoproteic content occurs.

Typically the therapeutic regimen of the present invention can envision concomitant administration of the two active ingredients CP and CPK or alternatively also sequential administration.

According to an embodiment a regimen of treatment is envisioned, including a first phase of administration of an effective amount of creatine phosphate (CP) and a second phase of administration of an effective amount of CPK. Typically the two administration phases are carried out within a short time, for example in the range between 1 min and 1 hour, and suitable for the two components (CP, CPK) to be both active at the same time in the site of action and so to be able to act synergistically on the atherosclerotic plaque.

According to an embodiment the regimen of treatment of the invention envisions the concomitant administration of CP and CPK for example using a pharmaceutical preparation including the two active ingredients in therapeutically effective amounts.

According to an embodiment, the treatment by means of the therapy of the present invention includes the administration of an amount of creatine phosphate (CP) 200 to 5000 mg, preferably 1000 to 1500 mg, even more preferably 1000 to 1200 mg in combination with the concomitant or sequential administration of creatine phosphokinase (CPK) 30 to 300 IU, preferably 40 to 200 IU daily.

Dose regimens can be adjusted in order to improve the therapeutic response through the administration at doses and for periods effective to reduce or eliminate the pathologic conditions. For example, CPK and CP can be administered once a day or in divided daily doses.

According to an embodiment of the invention a pharmaceutical composition is provided including an effective amount of CP and CPK in association with a pharmaceutically acceptable vehicle.

Typically CP and CPK are administered parenterally, typically intravenously, preferably by drip.

An embodiment concerns a pharmaceutical composition including at least one of CP or CPK in the form of powder, typically a lyophilized, ready to be dissolved in a suitable sterile solvent al the time of use.

Suitable solvents for use according to the invention include physiologically acceptable solvents such as aqueous physiological saline.

According to another embodiment, CP and CPK are formulated in a single pharmaceutical composition in selected weight ratios. For example the weight ratio CPK to CP is included in the range from 1:25 to 1:2500 and preferably from 1:300 to 1:500 and even more preferably is approximately 1:400.

Combined treatment with CP and CPK according to the present patent application determines a very quick efflux of triglycerides, even if the atherosclerotic plaque was treated only once. Then, the therapeutic treatment based on combined use of CP and CPK allows to obtain results already from the first administration, however to obtain a clinically significant lipolysis a multiplicity of administrations of CPK and CP in vivo is envisioned.

According to an embodiment, administration continues for some days since macrophages can mobilize triglycerides and lipoproteins markedly more quickly than free and esterified cholesterol. To be removed from the plaque, excreted cholesterol should be bound to an acceptor in the medium, such as HDL.

As an example, a slow and continuous intravenous infusion of a saline with CP, 5 g, dissolved therein can be given for 1 hour: during the infusion a single intravenous injection (bolus) of CPK (liquid), 200 IU is administered. The dose of CPK and CP, of course, depends on the obtained results and can variate. According to an embodiment, administered CPK is recombinant, to avoid the possibility of allergic reactions. In another embodiment, CPK is obtained by nanotechnology.

Use of exogenous CPK and CP can provide the necessary energy to activate the enzymes of the plaque and activate the biochemical activities of the plaque itself. Initially there is only efflux of triglycerides because the efflux occurred very quickly due to the addition of CP and CPK. In fact, macrophages mobilize triglycerides quickly, markedly more quickly than free and esterified cholesterol. The efflux of cholesterol from the plaque requires a much longer time.

According to an embodiment CP and CPK are administered to patients with atherosclerosis to reduce atherosclerotic plaques and stenosis of the artery and the administration of these active ingredients lasts some days, preferably once or twice daily. In an embodiment, excreted cholesterol should be bound to an acceptor in the medium, such as HDL, to be more effectively removed from the plaque.

It has been observed that concomitant or sequential administration of CP and CPK determines an efflux of triglycerides, lipoproteins and cholesterol from the atherosclerotic plaque. This phenomenon is highly significant to obtain a regression or resolution of the plaque. In fact blood levels of triglyceride-rich lipoproteins (VLDL and chylomicrons), are associated to the progression of atherosclerosis regardless of the levels of HDL cholesterol present in blood. Triglyceride-rich lipoproteins in general and VLDL in particular, have been identified inside plaques coming from people with atherosclerosis. The mechanism of development of atherosclerosis starting from high plasmatic levels of triglyceride-rich lipoproteins certainly involves transferring of these lipoproteins from the circulatory district, that is plasma, to the intima of the artery. Plasmatic lipoproteins can cross the endothelial barrier and enter in the arterial wall. At this point they can be directly phagocyted by macrophages to produce foam cells rich in lipids, the key type of cell of the initial atherosclerotic plaque and due to their massive accumulation, they promote the formation of atherosclerosis. Combined therapy with CP and CPK acts at these points determining a spontaneous efflux of TG and lipoproteins from the plaque. Typically this efflux requires some days and, in turn, it is quicker than the efflux of cholesterol. In ischemic tissues a marked decrease of ATP and CP due to ischemia and hypoxia has been observed: in atherosclerosis the existence of intraplaque ischemia and hypoxia with low enzyme activity is demonstrated. Similarly, the content of both ATP and CP in the plaque, results low. Use of exogenous CP and CPK can restart biochemical activities in the plaque itself. Administration of the association of CP and CPK to atherosclerotic patients determines the efflux of lipoproteins from the plaque reducing size and thickness of said plaque.

As recorded in the tests performed and described hereinafter, in ischemic tissues a marked decrease of ATP, CP and enzymatic activity has been observed as a result of ischemia and hypoxia. In atherosclerosis the existence of ischemia, hypoxia and low enzymatic intraplaque activity has been demonstrated. This is the reason why the intra-plaque content of ATP and CP is very low.

The following examples are provided merely by way of illustration of the present invention and should not be intended to be limiting.

### Example 1

### TESTS CARRIED OUT

Regarding the examples, the following abbreviations were used:
TC total cholesterol
HDL cholesterol high density lipoproteins
LDL cholesterol low density lipoproteins
VLDL cholesterol very low density lipoproteins
TG triglycerides
CP creatine phosphate
CPK-MM human muscular creatine phosphate kinase

To achieve the atherosclerotic plaque treatment regimen according to an embodiment of the present invention, many clinical tests were performed and accurate experimental studies were carried out.

To perform these investigations atherosclerotic plaques removed from internal carotid arteries of patients undergoing endarterectomy caused by carotid stenosis due to the plaque were used.

A single plaque was washed and then incubated for ten hours in Tyrode's solution (200 ml) at 37 °C bubbling oxygen 95% additioned with CO 5% through. CP is added for one hour (initially 200 mg, then 100 mg every six min to a total amount of 1200 mg). During the intervals 40 IU of CPK-MM are added. Concentrations of TG, HDL, LDL, TC in the medium are measured.

A quick and massive efflux of TG from the plaque in the medium appeared in the first hour and then only a slight increase with only traces of cholesterol.

Said efflux from the plaque to the medium is quick and caused by the effect of CP and CPK on the plaque itself. Spontaneous efflux of TG and lipoproteins from the plaque requires some days instead and, in turn, this is quicker than the efflux of cholesterol. In ischemic tissues a marked decrease of ATP and CP due to ischemia and hypoxia has been observed: in atherosclerosis the existence of intraplaque ischemia and hypoxia is demonstrated. Similarly, the content of both ATP and CP in the plaque results low.

### Example 2

Atherosclerotic plaques of internal carotid artery removed from 8 patients undergoing endarterectomy caused by carotid stenosis due to the plaque (stenosis 80-90%) were used. A plaque obtained immediately after surgical operation is placed in cold Tyrode's solution then is treated, from half an hour to an hour, after removal occurred with surgical operation. Plaques are washed and placed in Tyrode's solution (200 ml) and incubated for ten hours at 37 °C bubbling oxygen 95% additioned with CO 5% through. CP and CPK-MM from skeletal human muscle (purchased from Calbiochem), are added to the solution. CP is added beginning with 200 mg and then adding 100 mg every 6 min for 1 h (total 1200 mg): during the intervals 40 Ul of CPK-MM are added. Concentrations of TG, HDL, LDL, TC in the medium are measured by the use of the Hitachi enzymatic method (Roche diagnostic, Mannheim) at zero hours and 1 hour, 2 hours, 4 hours, 6 hours, 8 hours, 10 hours, after addition of CPK and CP. The lower limit of sensitivity (analytical sensitivity) of said enzymatic method is 3 mg/dl: concentrations lower than 3 mg/dl are not detectable. Half-life of CPK-MM is about 12 hours, but enzymatic activity is present until 23 hours with decreasing values. CPK-BB, present in brain, is unstable in human plasma quickly losing its activity that is reduced by the 50% in approximately 15 min. Half-life of CP is 4-7 min with subsequent decrease of value until 250 min. Atherosclerotic lesions are of severe type (type V).

### Clinical Recordings

Addition of CPK and CP to the solution determined a notable efflux of triglycerides from the plaque in the medium with only traces of cholesterol. Values of HDL, LDL and TC are undetectable since lower than 3 mg/ml. To examine the time of action of CPK and CP, levels of lipoproteins are measured for a period of 10 hours. Said efflux of TG occurs very quickly in the first hour; after that, only a slight increase (Table 1) occurs. Said quick and great efflux of TG is due to the addition of CPK and CP. Generally spontaneous efflux of TG and lipoproteins from macrophages (foam cells) requires some days and is however much quicker than the efflux of cholesterol. Histological examination of the lesions detects a thick connective tissue with hyaline with extensive calcifications but only some lipidic depositions. All the recorded data were schematically summarized in Table 1 here below.

**TABLE 1 Clinical recordings and lipoproteins**

| PATIENT | GENDER | AGE | TG mg/dl | | | | | | |
|---|---|---|---|---|---|---|---|---|---|
| | | | 0 h | 1^{st} h | 2^{nd} h | 4^{th} h | 6^{th} h | 8^{th} h | 10^{th} h |
| 1 | M | 74 | 0 | 216 | 218 | 220 | 221 | 223 | 224 |
| 2 | M | 76 | 0 | 179 | 182 | 183 | 185 | 186 | 188 |
| 3 | M | 70 | 0 | 176 | 181 | 183 | 185 | 185 | 186 |
| 4 | F | 79 | 0 | 208 | 210 | 211 | 212 | 212 | 213 |
| 5 | M | 79 | 0 | 198 | 197 | 200 | 203 | 204 | 205 |
| 6 | F | 78 | 0 | 501 | 503 | 504 | 505 | 508 | 509 |
| 7 | M | 78 | 0 | 510 | 508 | 507 | 512 | 517 | 520 |
| 8 | M | 69 | 0 | 254 | 235 | 232 | 238 | 240 | 242 |
| 8 | | | 0 | 280 | 279 | 280 | 282 | 284 | 285 |

As highlighted by data presented in Table 1, the effects of CPK and CP on the plaque are immediately visible and determine a notable efflux of triglycerides from the plaque to the medium with consequent reduction of the size of the plaque itself.

## Claims

1. Creatine phosphate (CP) and creatine phosphokinase (CPK) for combined use in the treatment of an atherosclerotic plaque present in the wall of an artery of a patient.

2. Creatine phosphate (CP) and creatine phosphokinase (CPK) for combined use according to claim 1 **characterized in that** CP and CPK are administered concomitantly.

3. Creatine phosphate (CP) and creatine phosphokinase (CPK for combined use according to claim 1 **characterized in that** CP is administered before CPK.

4. Creatine phosphate (CP) and creatine phosphokinase (CPK) for combined use according to any of claims 1-3, **characterized in that** creatine phosphate (CP) is administered at a dose of 1000 to 5000 mg, preferably of 1000 to 1200 mg.

5. Creatine phosphate (CP) and creatine phosphokinase (CPK) for combined use according to any of claims 1-4, **characterized in that** creatine phosphokinase (CPK) is administered at a dose of 40 to 200 IU.

6. Creatine phosphate (CP) and creatine phosphokinase (CPK) for combined use according to any of preceding claims 1-5 **characterized in that** creatine phosphate (CP) and creatine phosphokinase (CPK) are administered intravenously.

7. Creatine phosphate (CP) and creatine phosphokinase (CPK) for combined use according to any of preceding claims 1-5 **characterized in that** creatine phosphate (CP) is administered in the form of an injectable solution.

8. Creatine phosphate (CP) and creatine phosphokinase (CPK) for combined use according to any of preceding claims 1-7 **characterized in that** administration of creatine phosphate (CP) and creatine phosphokinase (CPK) is repeated for several days.

9. Creatine phosphate (CP) and creatine phosphokinase (CPK) for combined use according to any of preceding claims 1-8 **characterized in that** administration of creatine phosphate (CP) and creatine phosphokinase (CPK) is repeated several times, and once a day or several times in the same day.

10. Pharmaceutical composition comprising creatine phosphate (CP) and creatine phosphokinase (CPK) and a pharmaceutically acceptable vehicle for use in the treatment of an atherosclerotic plaque present in the endotelium of an artery of a patient.

11. Pharmaceutical composition for use according to claim 10 **characterized in that** it is in the form of a sterile injectable saline solution.

## Patentansprüche

1. Kreatinphosphat (CP) und Kreatin-Phosphokinase (CPK) zur kombinierten Verwendung für die Behandlung einer arteriosklerotischen Plaque die sich in der Arterienwand eines Patienten befindet.

2. Kreatinphosphat (CP) und Kreatin-Phosphokinase (CPK) zur kombinierten Verwendung nach Anspruch 1, **dadurch gekennzeichnet dass** CP und CPK gleichzeitig verabreicht werden.

3. Kreatinphosphat (CP) und Kreatin-Phosphokinase (CPK) zur kombinierten Verwendung nach Anspruch 1, **dadurch gekennzeichnet dass** CP vor CPK verabreicht wird.

4. Kreatinphosphat (CP) und Kreatin-Phosphokinase (CPK) zur kombinierten Verwendung nach einem der Ansprüche 1-3, **dadurch gekennzeichnet dass** Kreatinphosphat (CP) in einer Dosis von 1000 bis 5000 mg, bevorzugt von 1000 bis 1200 mg, verabreicht wird.

5. Kreatinphosphat (CP) und Kreatin-Phosphokinase (CPK) zur kombinierten Verwendung nach einem der Ansprüche 1-4, **dadurch gekennzeichnet dass** Kreatin-Phosphokinase (CPK) in einer Dosis von 40 bis 200 IU verabreicht wird.

6. Kreatinphosphat (CP) und Kreatin-Phosphokinase (CPK) zur kombinierten Verwendung nach einem der vorherhegenden Ansprüche 1-5, **dadurch gekennzeichnet dass** Kreatinphosphat (CP) und Kreatin-Phosphokinase (CPK) intravenös verabreicht werden.

7. Kreatinphosphat (CP) und Kreatin-Phosphokinase (CPK) zur kombinierten Verwendung nach einem der vorherhegenden Anspruche 1-5, **dadurch gekennzeichnet dass** Kreatinphosphat (CP) in Form einer injizierbaren Lösung verabreicht wird.

8. Kreatinphosphat (CP) und Kreatin-Phosphokinase (CPK) zur kombinierten Verwendung nach einem der vorherhegenden Ansprüche 1-7, **dadurch gekennzeichnet dass** die Verabreichung von Kreatinphosphat (CP) und Kreatin-Phosphokinase (CPK) für mehrere Tage wiederholt wird.

9. Kreatinphosphat (CP) und Kreatin-Phosphokinase (CPK) zur kombinierten Verwendung nach einem der vorherhegenden Ansprüche 1-8, **dadurch gekennzeichnet dass** die Verabreichung von Kreatinphosphat (CP) und Kreatin-Phosphokinase (CPK) mehrmals und einmal am Tag oder mehrmals am selben Tag wiederholt wird.

10. Pharmazeutische Zusammensetzung umfassend Kreatinphosphat (CP) und Kreatin-Phosphokinase (CPK) und einen pharmazeutisch annehmbaren Träger zur Verwendung für die Behandlung einer arteriosklerotischen Plaque die sich im Endothelium einer Ader eines Patienten befindet.

11. Pharmazeutische Zusammensetzung zur Verwendung nach Anspruch 10, **dadurch gekennzeichnet dass** sie in Form einer sterilen injizierbare Salzlösung ist.

## Revendications

1. Phosphocréatine (PCr) et créatine phosphokinase (CPK) pour l'utilisation combinée dans le traitement d'une plaque d'athérome présente dans la paroi d'une artère d'un patient.

2. Phosphocréatine (PCr) et créatine phosphokinase (CPK) pour l'utilisation combinée selon la revendication 1, **caractérisées en ce que** PCr et CPK sont administrées concomitamment.

3. Phosphocréatine (PCr) et créatine phosphokinase (CPK) pour l'utilisation combinée selon la revendication 1, **caractérisées en ce que** PCr est administrée avant de CPK.

4. Phosphocréatine (PCr) et créatine phosphokinase (CPK) pour l'utilisation combinée selon quelconque des revendications 1-3, **caractérisées en ce que** phosphocréatine (PCr) est administrée à un dosage de 1000 à 5000 mg, de préférence de 1000 à 1200 mg.

5. Phosphocréatine (PCr) et créatine phosphokinase (CPK) pour l'utilisation combinée selon quelconque des revendications 1-4, **caractérisées en ce que** créatine phosphokinase (CPK) est administrée à un dosage de 40 à 200 lU.

6. Phosphocréatine (PCr) et créatine phosphokinase (CPK) pour l'utilisation combinée selon quelconque des revendications 1-5 précédentes, **caractérisées en ce que** phosphocréatine (PCr) et créatine phosphokinase (CPK) sont administrées par voie intraveineuse.

7. Phosphocréatine (PCr) et créatine phosphokinase (CPK) pour l'utilisation combinée selon quelconque des revendications 1-5 précédentes, **caractérisées en ce que** phosphocréatine (PCr) est administrée sous forme d'une solution injectable.

8. Phosphocréatine (PCr) et créatine phosphokinase (CPK) pour l'utilisation combinée selon quelconque des revendications 1-7 précédentes, **caractérisées en ce que** l'administration de phosphocréatine (PCr) et de créatine phosphokinase (CPK) est répétée pour plusieurs jours.

9. Phosphocréatine (PCr) et créatine phosphokinase (CPK) pour l'utilisation combinée selon quelconque des revendications 1-8 précédentes, **caractérisées en ce que** l'administration de phosphocréatine (PCr) et de créatine phosphokinase (CPK) est répétée plusieurs fois et une fois au jour ou plusieurs fois dans le même jour.

10. Composition pharmaceutique qui comprend phosphocréatine (PCr) et créatine phosphokinase (CPK) et un véhicule pharmaceutiquement acceptable pour l'utilisation dans le traitement d'une plaque d'athérome présente dans l'endothélium d'une artère d'un patient.

11. Composition pharmaceutique per l'utilisation selon la revendication 10, **caractérisée en ce qu'**elle est sous forme d'une solutionne saline injectable stérile.
